# EUROPEAN PATENT SPECIFICATION

(11) **EP 1 307 147 B1**
(45) Date of publication and mention of the grant of the patent: **06.10.2010**
(21) Application number: 01962018.6
(22) Date of filing: 10.08.2001
(51) Int. Cl.: A61B 17/12, C21D 1/42, C21D 9/02

(54) **VARIABLE SOFTNESS VASO-OCCLUSIVE COILS**
GEFÄSSVERSCHLIESSENDE SPIRALEINRICHTUNG MIT VARIABLER WEICHHEIT
SPIRALES D'OCCLUSION VASCULAIRE A SOUPLESSE VARIABLE

(30) Priority: 11.08.2000 US 637470
(43) Date of publication of application: 07.05.2003
(73) Proprietor: Boston Scientific Scimed, Inc., Maple Grove, MN 55311 (US)
(72) Inventor: TEOH, Clifford, Los Altos, CA 94024-6114 (US)
(74) Representative: Vossius & Partner
(86) International application number: PCT/US2001/025008
(87) International publication number: WO 2002/013706

(56) References cited:
- EP-A- 0 820 726
- DE-C- 19 942 811
- US-A- 2 306 925
- US-A- 5 957 948

## Description

### FIELD OF THE INVENTION

This invention is in the field of an implantable vaso-occlusive device. In particular, the invention relates to deployable devices, for example coils, that efficiently self-form into a three-dimensional shape when deployed into a body cavity and methods of producing the same. Without changing overall dimensions, coils can be made to be softer in selective zones, thereby promoting efficient coil formation into desired three-dimensional shapes.

### BACKGROUND

Vaso-occlusion devices are surgical implements or implants that are placed within the vasculature of the human body, typically via a catheter, either to block the flow of blood through a vessel making up that portion of the vasculature through the formation of an embolus or to form such an embolus within an aneurysm stemming from the vessel. One widely used vaso-occlusive device is a helical wire coil having windings which may be dimensioned to engage the walls of the vessels. Other less stiff helically coiled devices have been described, as well as those involving woven braids.

For instance, U.S. Pat. No. 4,994,069, to Ritchart et al., describes a vaso-occlusive coil that assumes a linear helical configuration when stretched and a folded, convoluted configuration when relaxed. The stretched condition is used in placing the coil at the desired site (by its passage through the catheter) and the coil assumes a relaxed configuration--which is better suited to occlude the vessel--once the device is so placed. Ritchart et al. describes a variety of shapes. The secondary shapes of the disclosed coils include "flower" shapes and double vortices. A random shape is described, as well.

Vaso-occlusive coils having attached fibrous elements in a variety of secondary shapes are shown in U.S. Pat. No. 5,304,194, to Chee et al. Chee et al. describes a helically wound device having a secondary shape in which the fibrous elements extend in a sinusoidal fashion down the length of the coil. These coils, as with Ritchart et al., are produced in such a way that they will pass through the lumen of a catheter in a generally straight configuration and, when released from the catheter, form a relaxed or folded shape in the lumen or cavity chosen within the human body. The fibrous elements shown in Chee et al. enhance the ability of the coil to fill space within the vasculature and to facilitate formation of embolus and subsequent allied tissue.

Vaso-occlusive coils having little or no inherent secondary shape have also been described. For instance, co-owned U.S. Patent Numbers 5,690,666 and 5,826,587 by Berenstein et al., describes coils having little or no shape after introduction into the vascular space.

There are a variety of ways of discharging shaped coils and linear coils into the human vasculature. In addition to those patents which apparently describe only the physicals pushing of a coil out into the vasculature (e.g., Ritchart et al.), there are a number of other ways to release the coil at a specifically chosen time and site. U.S. Pat. No. 5,354,295 and its parent, U.S. Pat. No. 5,122,136, both to Guglielmi et al., describe an electrolytically detachable embolic device.

A variety of mechanically detachable devices are also known. For instance, U.S. Pat. No. 5,234,437, to Sepetka, shows a method of unscrewing a helically would coil from a pusher having interlocking surfaces. U.S. Pat. No. 5,250,071, to Palermo, shows an embolic coil assembly using interlocking clasps mounted both on the pusher and on the embolic coil. U.S. Pat. No. 5,261,916, to Engelson, shows a detachable pusher-vaso-occlusive coil assembly having an interlocking ball and keyway-type coupling. U.S. Pat. No. 5,304,195, to Twyford et al., shows a pusher-vaso-occlusive coil assembly having an affixed, proximally extending wire carrying a ball on its proximal end and a pusher having a similar end. The two ends are interlocked and disengage when expelled from the distal tip of the catheter. U.S. Pat. No. 5,312,415, to Palermo, also shows a method for discharging numerous coils from a single pusher by use of a guidewire which has a section capable of interconnecting with the interior of the helically wound coil. U.S. Pat. No. 5,350,397, to Palermo et al., shows a pusher having a throat at its distal end and a pusher through its axis. The pusher sheath will hold onto the end of an embolic coil and will then be released upon pushing the axially placed pusher wire against the member found on the proximal end of the vaso-occlusive coil.

US-A-2,306,925 relates to electrodes for electric discharge devices, and to the construction and fabrication of such electrodes, especially cathodes. The method for fabrication such electrodes comprises the step of winding relatively fine wire on a composite mandrel which comprises in its cross-section segregated materials of different character, and the step of subsequently eliminating one of the said mandrels materials, so that the remaining mandrel material only partially fills the convolutions of relatively fine wire.

None of these documents disclose coils with enhanced areas of softness to promote early coil formation or methods of producing and using these selectively softer coils.

### SUMMARY OF THE INVENTION

Thus, this invention includes a composite mandrel according to claim 1 useful in making vaso-occlusive devices and a method of using the mandrel as defined in claim 6.

In one aspect, the invention includes a composite mandrel comprising one or more conductive materials and one or more non-conductive material. The conductive material (*e.g.*, one or more metallic materials such as ferritic steel or copper) and non-conductive material (*e.g.,* ceramic) are distinct and non-overlapping. There are multiple areas of conductive material and multiple areas of non-conductive material in a composite mandrel according to the present invention. The composite mandrels may be a variety of shapes including cylindrical, spherical, rectangular, square, etc. Further, the shape of the composite mandrel can be altered after a wire is wound about it.

In a further aspect, the invention includes a method of making a variable softness vaso-occlusive device comprising winding an elongated wire about any of the composite mandrels described herein and inductively heating the wire and mandrel. In certain embodiments, the composite mandrel (*e.g.*, composite of ceramic and ferritic steel) is linear, for example a cylinder. In other embodiments, the composite mandrel is another three dimensional shape, for example a sphere, square, cube, etc. In yet other embodiments, a cylindrical mandrel is used to wind the wire and, after winding, the mandrel is shaped into a different three-dimensional configuration for winding.

These and other embodiments of the subject invention will readily occur to those of skill in the art in light of the disclosure herein.

### BRIEF DESCRIPTION OF THE DRAWINGS

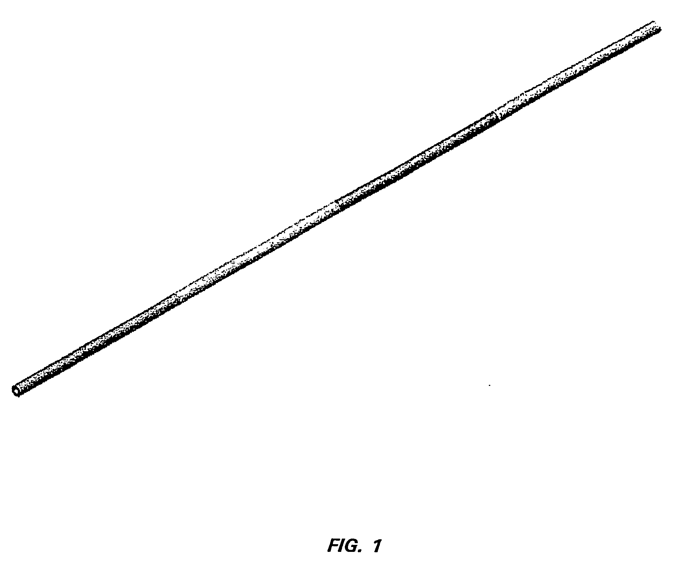

Figure 1 depicts a mandrel made of alternating areas of conductive material and non-conductive material.

### DESCRIPTION OF THE INVENTION

Vaso-occlusive devices having selective zones of relative softness are described herein. The invention includes composite mandrels (*e.g.*, composites of conductive and non-conductive materials) useful in producing the selectively soft vaso-occlusive devices. Methods of making these devices also form an aspect of this invention.

Advantages of the present invention include, but are not limited to, (i) more easily deployable coils; (ii) less rotation of the primary coil upon deployment (*e.g.*, upon deployment from a catheter into a body cavity); and (iii) rapid formation of three dimensional structures upon deployment into a body cavity; all without the necessity of varying the diameter of the wire making up the coil.

It must be noted that, as used in this specification and the appended claims, the singular forms "a", "an", and "the" include plural referents unless the content clearly dictates otherwise. Thus, for example, reference to "a coil" includes a mixture of two or more such devices and the like.

The vaso-occlusive devices described herein are typically formed by winding a conductive wire (*e.g.*, a metallic wire) around a mandrel, for example winding the wire into a first helix and then winding the first helix into a secondary form. The pattern of winding on the composite mandrel provides both the three dimensional shape of the invention at deployment and also determines which areas of the coil are in contact with which areas of the mandrel. For instance, the wire can be wound into a primary helix on a linear (*e.g.*, cylindrical) composite mandrel, annealed and then wound again on the same or differently three-dimensional shaped composite mandrel. Alternatively, the wire can be wound around a linear (*e.g.*, cylindrical) composite mandrel and, after winding, the wire-mandrel complex can be shaped into a different (*e.g.*, non-cylindrical) three dimensional configuration prior to annealing.

The assembly of mandrel and coil is typically heat treated, preferably by induction. The secondary form is one which, when ejected from a delivery catheter, forms a generally three-dimensional shape, filling first the outer periphery of the three-dimensional shape and then the center region. Desirably, the vaso-occlusive device is of a size and shape suitable for fitting snugly within a vascular cavity (*e.g.*, an aneurysm, or perhaps, a fistula).

Suitable winding mandrels may be a variety of shapes (*e.g.*, cylindrical, square, spherical, circular, rectangular, etc.) and may be solid or hollow. Some exemplary shapes of mandrels are shown in co-owned U.S. Patent No. 5,957,948 to Mariant et al. As noted above, the winding mandrel should be of sufficient heat resistance to allow a moderate annealing step. Further, also as noted above, after winding of the wire, the shape of the mandrel may be altered (*e.g.*, cylinder to sphere or other three-dimensional shape). The mandrel may be made of a refractory material such as glass, borosilicate, alumina or zirconia (for heat-treating devices made of purely metallic components) or may be made of one or more metallic materials (*e.g.*, stainless steel, ferritic steel, copper, etc.).

The mandrel is a composite made from one or more conductive materials and one or more non-conductive materials. Conductivity (also known as specific conductance or electrical conductivity) refers to the ratio of the electric current density to the electric field in a given material. Thus, conductivity is the ability of a substance to conduct an electric current. Non-conductive materials (*e.g.*, insulators) conduct little or no electricity. Non-limiting examples of non-conductive materials include ceramic, wood or plastic materials. Conductive materials, which are able to conduct electrical materials, include but are not limited to materials such as metals (*e.g.*, ferritic steel).

The conductive areas of the mandrel may overlap with the non-conductive material or, alternatively, there may be no overlap (*e.g.*, the conductive material and non-conductive areas of the mandrel are distinct). In addition, variable softness coils can be prepared by adding varying amounts of non-conductive material to the conductive material. Similarly, varying amounts of conductive material can be added to the non-conductive zones of the mandrel. Shown in FIG 1 is one embodiment where the mandrel is made of alternating zones of at least one conductive material and at least one non-conductive material. Thus, as will be readily apparent to one skilled in the art from the teachings herein, the size and position of the various areas of conductive material can be varied to produce coils having differing zones of softness. Depending on their position, the zones of softness promote earlier formation of the secondary configuration upon deployment into a body cavity (*e.g.*, an aneurysm). One of skill in the art could readily determine both the nature of the mandrel and the winding pattern useful to achieve the desired coil type. For example, the entire distal portion of a coil can be made softer by using the appropriate mandrel and heating step.

The assembly of wire and composite mandrel is annealed, preferably by heating inductively. Induction heating is widely used in industrial processes such as metal hardening, preheating, brasing or thixoforming. It consists in generating heat info a workpiece by means of the Joule effect resulting from eddy currents induced by a pulsating magnetic field. A typical annealing step for a platinum/tungsten alloy coil would involve a inductive heating step for between about 15-20 minutes to about 6 hours at a temperature of between about 537,78°C (1000°F) and about 815,56°C (1500°F). The primary coil is typically linear after it has been wound and heat treated. Moreover, the areas of the coil wound about the conductive material portions of the mandrel will be softer than the areas of the coil wound around the non-conductive portions of the mandrel.

Induction heating of the wire and composite mandrel produces a devices with selected areas of enhanced softness. Softness may be measured by any test know in the art, for example, "tip buckling" tests, bending stiffness tests, and/or compression tests. Tip buckling is tested using a balance to measure the force transmitted to the transducer with the device oriented normal to the transducer surface. Bending stiffness tests are similar to standard cantilevered tests, but for coil applications which have a secondary shape or helix, a semi-circular section of the coil is held in place for bending stiffness measurements. Compression tests are also done in the same manner but a rounded surface is used to measure the compressive resistance the coil exerts. Other tests for softness and hardness suitable for use in vaso-occlusive devices will be known to those of skill in the art. The variable softness coils of the present invention will typically include at least one area that is 2 to 10 fold (or any integer in between), preferably 5 to 50 fold (or any integer in between), even more preferably, 5 to 500 fold (or any integer in between) softer than the hardest areas of the device as measured by one or more softness tests. Coils known in the art currently deflect about 5% of full scale or approximately a single digit inch/gram. It will be readily apparent that different areas in the coil may have differing degrees of softness, relative to the hardest areas.

The material used in constructing the vaso-occlusive member having enhanced areas of softness may be any of a wide variety of materials; preferably, the wire is a radio-opaque material such as a metal. Suitable metals and alloys for the wire making up the primary coil include the Platinum Group metals, especially platinum, rhodium, palladium, rhenium, as well as tungsten, gold, silver, tantalum, and alloys of these metals. These metals have significant radiopacity and in their alloys may be tailored to accomplish an appropriate blend of flexibility and stiffness. They are also largely biologically inert. Highly preferred is a platinum/tungsten alloy.

The wire may also be of any of a wide variety of stainless steels if some sacrifice of radiopacity may be tolerated. Very desirable materials of construction, from a mechanical point of view, are materials which maintain their shape despite being subjected to high stress. Certain "super-elastic alloys" include nickel/titanium alloys (48-58 atomic % nickel and optionally containing modest amounts of iron); copper/zinc alloys (38-42 weight % zinc); copper/zinc alloys containing 1-10 weight % of beryllium, silicon, tin, aluminum, or gallium; or nickel/aluminum alloys (36-38 atomic % aluminum). Particularly preferred are the alloys described in U.S. Pat. Nos. 3,174,851; 3,351,463; and 3,753,700. Especially preferred is the titanium/nickel alloy known as "nitinol". These are very sturdy alloys which will tolerate significant flexing without deformation even when used as a very small diameter wire. If a superelastic alloy such as nitinol is used in the device, the diameter of the coil wire may be significantly smaller than that used when the relatively more ductile platinum or platinum/tungsten alloy is used as the material of construction.

Generally speaking, when the device is formed of a metallic coil and that coil is a platinum alloy or a superelastic alloy such as nitinol, the diameter of the wire used in the production of the coil will be in the range of 0.0013 (0.0005) and 0.0152 cm (0.006 inches). The wire of such diameter is typically then wound into a primary coil having a primary diameter of between 0.0127 (0.005) and 0.0889 cm (0.035 inches). For most neurovascular indications, the preferable diameter is 0.025 (0.010) to 0.0457 cm (0.018 inches). We have generally found that the wire may be of sufficient diameter to provide a hoop strength to the resulting device sufficient to hold the device in place within the chosen body cavity without distending the wall of the cavity and without moving from the cavity as a result of the repetitive fluid pulsing found in the vascular system.

The axial length of the primary coil will usually fall in the range of 0.5 to 100 cm, more usually 2.0 to 40 cm. Depending upon usage, the coil may well have 100-400 turns per centimeter, preferably 200-300 turns per centimeter. All of the dimensions here are provided only as guidelines and are not critical. However, only dimensions suitable for use in occluding sites within the human body are included in the scope of this invention.

The overall diameter of the device as deployed is generally between 2 and 20 millimeters. Most aneurysms within the cranial vasculature can be treated by one or more devices having those diameters. Of course, such diameters are not a critical aspect.

Thus, although the coil material is preferably conductive, the devices may include some radiolucent fibers or polymers (or metallic threads coated with radiolucent or radiopaque fibers) such as Dacron (polyester), polyglycolic acid, polylactic acid, fluoropolymers (polytetrafluoro-ethylene), Nylon (polyamide), or even silk. Polymer materials may be filled with some amount of a known radiopaque material such as powdered tantalum, powdered tungsten, bismuth oxide, barium sulfate, and the like. Also contemplated is the attachment of various fibrous materials to the coil for the purpose of adding thrombogenicity to the resulting assembly. The fibrous materials may be attached in a variety of ways. A series of looping fibers may be looped through or tied to coil and continue axially down the coil. Another variation is by tying the tuft to the coil. Tufts may be tied at multiple sites through the coil to provide a vast area of embolus forming sites. The primary coil may be covered by a fibrous braid. The method for producing the former variation is described in U.S. Pat. Nos. 5,226,911 and 5,304,194 to Chee. The method of producing the fibrous braid is described in U.S. Pat. No. 5,382,259, issued Jan. 17, 1995, to Phelps and Van.

The coils described herein can also include additional additives, for example, any material that exhibits biological activity *in vivo.* Non-limiting examples of suitable bioactive materials are known to those of skill in the art. Preferably, these materials are added after annealing.

The coils described herein may be associated with other materials, such as radioactive isotopes, bioactive coatings, polymers, fibers, etc., for example by winding, braiding or coating onto the device one or more of these materials, typically prior to introduction into the subject. Methods of associating polymeric materials with a solid substrate such as a coil are known to those of skill in the art, for example as described in U.S. Patent Nos. 5,522,822 and 5,935,145. In yet other embodiments, the solid substrate itself is made to be radioactive for example using radioactive forms of the substrate material (*e.g.*, metal or polymer). Thus, the solid substrates can be made to be radioactive after formation by deposition (*e.g.*, coating, winding or braiding), impregnantion (*e.g.*, ion-beam or electrodeposition) or other techniques of introducing or inducing radioactivity.

In addition one or more metals, the occlusive devices may optionally include a wide variety of synthetic and natural polymers, such as polyurethanes (including copolymers with soft segments containing esters, ethers and carbonates), ethers, acrylates (including cyanoacrylates), olefins (including polymers and copolymers of ethylene, propylene, butenes, butadiene, styrene, and thermoplastic olefin elastomers), polydimethyl siloxane-based polymers, polyethyleneterephthalate, cross-linked polymers, non-cross linked polymers, rayon, cellulose, cellulose derivatives such nitrocellulose, natural rubbers, polyesters such as lactides, glycolides, caprolactones and their copolymers and acid derivatives, hydroxybutyrate and polyhydroxyvalerate and their copolymers, polyether esters such as polydioxinone, anhydrides such as polymers and copolymers of sebacic acid, hexadecandioic acid and other diacids, orthoesters may be used. In a preferred embodiment, the polymeric filament comprises the materials of the present invention or other suture materials that have already been approved for use in wound heating in humans.

### Methods of Use

The coils prepared by winding an elongated wire around a composite mandrel and heat treating the mandrel-coil assembly as described above are typically removed from the winding mandrel and loaded into a carrier for introduction into the delivery catheter. The devices are preferably first introduced to the chosen site using the procedure outlined below. This procedure may be used in treating a variety of maladies. For instance, in treatment of an aneurysm, the aneurysm itself may be filled with the mechanical devices prior to introducing the inventive composition. Shortly after the devices are placed within the aneurysm, an emboli begins to form and, at some later time, is at least partially replaced by neovascularized collagenous material formed around the vaso-occlusive devices.

In using the occlusive devices, a selected site is reached through the vascular system using a collection of specifically chosen catheters and guide wires. It is clear that should the site be in a remote site, e.g., in the brain, methods of reaching this site are somewhat limited. One widely accepted procedure is found in U.S. Patent No. 4,994,069 to Ritchart, et al. It utilizes a fine endovascular catheter such as is found in U.S. Patent No. 4,739,768, to Engelson. First of all, a large catheter is introduced through an entry site in the vasculature. Typically, this would be through a femoral artery in the groin. Other entry sites sometimes chosen are found in the neck and are in general well known by physicians who practice this type of medicine. Once the introducer is in place, a guiding catheter is then used to provide a safe passageway from the entry site to a region near the site to be treated. For instance, in treating a site in the human brain, a guiding catheter would be chosen which would extend from the entry site at the femoral artery, up through the large arteries extending to the heart, around the heart through the aortic arch, and downstream through one of the arteries extending from the upper side of the aorta. A guidewire and neurovascular catheter such as that described in the Engelson patent are then placed through the guiding catheter as a unit. Once the tip of the guidewire reaches the end of the guiding catheter, it is then extended using fluoroscopy, by the physician to the site to be treated using the vaso-occlusive devices. During the trip between the treatment site and the guide catheter tip, the guidewire is advanced for a distance and the neurovascular catheter follows. Once both the distal tip of the neurovascular catheter and the guidewire have reached the treatment site, and the distal tip of that catheter is appropriately situated, e.g., within the mouth of an aneurysm to be treated, the guidewire is then withdrawn. The neurovascular catheter then has an open lumen to the outside of the body. The devices are then pushed through the lumen to the treatment site. They are held in place variously because of their shape, size, or volume. These concepts are described in the Ritchart et al patent as well as others. Once the vaso-occlusive devices are situated in the vascular site, the embolism forms.

The mechanical or solid vaso-occlusion device may be used as a kit with the polymeric composition.

Modifications of the procedure and device described above, and the methods of using them will be apparent to those having skill in this mechanical and surgical art.

## Claims

1. A composite mandrel comprising one or more conductive materials and one or more non-conductive materials, wherein there are multiple areas of said conductive material and multiple areas of said non-conductive material and wherein the multiple areas of said conductive material are distinct from the multiple areas of said non-conductive material.

2. The mandrel of claim 1, wherein the conductive material and non-conductive material are distinct.

3. The mandrel of any one of claims 1 to 2, wherein the non-conductive material comprises a ceramic material.

4. The mandrel of any one of claims 1 to 2, wherein the conductive materials comprise one or more metallic materials.

5. The mandrel of claim 4, wherein the metallic material comprises ferritic steel.

6. A method of making a variable softness vaso-occlusive device comprising
(a) winding an elongated wire about the mandrel of any one of claims 1 to 5; and
(b) inductively heating the wire and mandrel.

7. The method of claim 6, wherein the conductive material of the mandrel comprises a metallic material.

8. The method of claim 7, wherein the metallic material comprises ferritic steel.

9. The method of claim 6, wherein the non-conductive material comprises a ceramic material.

10. The method of claim 6, wherein the mandrel is linear.

11. The method of claim 6, wherein the mandrel is a three-dimensional shape.

## Patentansprüche

1. Verbunddorn mit einem oder mehreren leitenden Materialien und einem oder mehreren nichtleitenden Materialien, wobei mehrere Bereiche aus dem leitenden Material und mehrere Bereiche aus dem nichtleitenden Material vorhanden sind und wobei sich die mehreren Bereiche aus dem leitenden Material von den mehreren Bereichen aus dem nichtleitenden Material unterscheiden.

2. Dorn nach Anspruch 1, wobei sich das leitende Material und nichtleitende Material unterscheiden.

3. Dorn nach einem der Ansprüche 1 bis 2, wobei das nichtleitende Material ein Keramikmaterial aufweist.

4. Dorn nach einem der Ansprüche 1 bis 2, wobei die leitenden Materialien ein oder mehrere metallische Materialien aufweisen.

5. Dorn nach Anspruch 4, wobei das metallische Material ferritischen Stahl aufweist.

6. Verfahren zur Herstellung einer Vasookklusionsvorrichtung mit variabler Weichheit mit:
(a) Wickeln eines länglichen Drahts um den Dorn nach einem der Ansprüche 1 bis 5; und
(b) induktives Erwärmen des Drahts und Dorns.

7. Verfahren nach Anspruch 6, wobei das leitende Material des Dorns ein metallisches Material aufweist.

8. Verfahren nach Anspruch 7, wobei das metallische Material ferritischen Stahl aufweist.

9. Verfahren nach Anspruch 6, wobei das nichtleitende Material ein Keramikmaterial aufweist.

10. Verfahren nach Anspruch 6, wobei der Dorn geradlinig ist.

11. Verfahren nach Anspruch 6, wobei der Dorn eine dreidimensionale Form hat.

## Revendications

1. Mandrin composite comprenant un ou plusieurs matériaux conducteurs et un ou plusieurs matériaux non conducteurs, dans lequel se trouvent de multiples zones dudit matériau conducteur et de multiples zones dudit matériau non conducteur et dans lequel les zones multiples dudit matériau conducteur sont distinctes des zones multiples dudit matériau non conducteur.

2. Mandrin selon la revendication 1, dans lequel le matériau conducteur et le matériau non conducteur sont distincts.

3. Mandrin selon l'une quelconque des revendications 1 à 2, dans lequel le matériau non conducteur comprend un matériau céramique.

4. Mandrin selon l'une quelconque des revendications 1 à 2, dans lequel les matériaux conducteurs comprennent un ou plusieurs matériaux métalliques.

5. Mandrin selon la revendication 4, dans lequel le matériau métallique comprend de l'acier ferritique.

6. Procédé de fabrication d'un dispositif d'occlusion vasculaire à souplesse variable comprenant
(a) l'enroulement d'un fil allongé autour du mandrin selon l'une quelconque des revendications 1 à 5 ; et
(b) le chauffage par induction du fil et du mandrin.

7. Procédé selon la revendication 6, dans lequel le matériau conducteur du mandrin comprend un matériau métallique.

8. Procédé selon la revendication 7, dans lequel le matériau métallique comprend de l'acier ferritique.

9. Procédé selon la revendication 6, dans lequel le matériau non conducteur comprend un matériau céramique.

10. Procédé selon la revendication 6, dans lequel le mandrin est linéaire.

11. Procédé selon la revendication 6, dans lequel le mandrin est de forme tridimensionnelle.
